(19) Europäisches Patentamt
European Patent Office
Office européen des brevets

(11) Publication number: **0 091 519**
**B1**

(12) **EUROPEAN PATENT SPECIFICATION**

(45) Date of publication of patent specification: **24.06.87**

(51) Int. Cl.$^4$: **A 61 K 31/13,** A 61 K 9/00

(21) Application number: **82301874.2**

(22) Date of filing: **08.04.82**

# (54) Topical acylaminophenols.

(43) Date of publication of application:
**19.10.83 Bulletin 83/42**

(45) Publication of the grant of the patent:
**24.06.87 Bulletin 87/26**

(84) Designated Contracting States:
**AT DE GB IT**

(56) References cited:
**FR-M- 2 342**
**FR-M- 7 624**
**US-A-2 653 158**
**US-A-3 551 462**
**US-A-4 329 366**
**CHEMICAL ABSTRACTS, vol. 72, no. 15, 13th April 1970, page 177, no. 76596f, Columbus Ohio (USA); A.JANCSO-GABOR et al.: "Irreversible impairment of thermoregulation induced by capsaicin and similar pungent substances in rats and guinea pigs"**
**CHEMICAL ABSTRACTS, vol. 86, no. 14, 4th April 1977, page 370, no. 95288n, Columbus Ohio (USA); J.BINA et al.: "Toxicological and dermatological problems in the cable industry".**

(73) Proprietor: **Johnson & Johnson Products Inc.**
**501 George Street**
**New Brunswick New Jersey 08903 (US)**

(72) Inventor: **Nashed, Wilson**
**1306 Jackson Drive**
**North Brunswick New Jersey (US)**
Inventor: **Rovee, David T.**
**30 Chelsea Way**
**Bridgewater New Jersey (US)**
Inventor: **Gander, Robert J.**
**Clark Lane**
**Whitehouse New Jersey (US)**

(74) Representative: **Jones, Alan John et al**
**CARPMAELS & RANSFORD**
**43 Bloomsbury Square**
**London, WC1A 2RA (GB)**

Note: *Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).*

**EP 0 091 519 B1**

Courier Press, Leamington Spa, England.

**Description**

Technical Field

This invention relates to topical pharmaceutical compositions adapted for only topical, to the exclusion of oral and injectable, administration, for the treatment of an inflammatory condition, comprising a pharmaceutically acceptable topical vehicle containing an anti-inflammatory agent comprising an N-acylaminophenol containing a normal aliphatic side chain having from six to twelve carbon atoms, in an amount sufficient to combat said inflammatory condition, and also its use for the manufacture of a medicament having the above activity.

There are many steroidal drugs suitable for treating particular responsive dermatological disorders. They have a broad range of applicable biological activities (e.g. cell membrane stabilization, vasoconstrictor activity, anti-mitotic effect, suppression of DNA and protein synthesis). However, steroids often exhibit undesired local and systemic side effects when used for prolonged periods. These may be manifested in the form of local skin atrophy, or in adrenal atrophy in the most severe case. Furthermore, a reduction in host defense mechanisms to infection may accompany their use.

Beyond the potential undesired side effects, steroids do not interact with the complete biological spectrum of inflammation. For example, ultraviolet (UV) light induced erythema is not amenable to treatment by steroidal anti-inflammatories. UV-induced early changes in skin such as vasodilation are related to the conversion of arachidonic acid to E prostaglandins or to intermediate forms such as HETE (12L-hydroxy-5,8,10,14-eicosatetraenoic acid) or the endoperoxides which are vasoactive and are believed to have cutaneous activity, such as triggering hyperproliferative epidermal activity. See, for example, Bem, J.L. and Greaves, M. W. 1974 Prostaglandin $E_1$ effects on epidermal cell growth "in vitro." *Arch. Derm. Forsch. 251*:35—41; Snyder, D. S. and Eaglstein, W. H. 1974 Topical indomethacin and sunburn. *Brit. J. Derm.* 90:91—93; Snyder, D. S. and Eaglstein, W. H. 1974 Intradermal anti-prostaglandin agents and sunburn. *J. Invest. Derm.* 62.47—50; and Goldyne, M. E. et al. 1973 Prostaglandin activity in human cutaneous inflammation: Detection by radio-immunoassay. *Prostaglandins* 4:737—749. See also Hsia, S. L., Ziboh, V. A. and Snyder, D. S. 1974. Naturally occurring and synthetic inhibitors of prostaglandin synthetase of the skin. *Prostaglandin Synthetase Inhibitors* 353—361. Although it is not understood completely, it is thought that these effects related to prostaglandin biosynthesis are important components of many dermatopathologies, so a drug which will interfere with this biosynthesis should be useful in the clinical improvement of the disease.

There are many non-steroidal compounds or agents which also have anti-inflammatory effects. Many of these are belived to act by blocking the prostaglandin synthetase complex of enzymes that are present in normal skin and are necessary for the biosynthetic processes described above. Furthermore, as a general rule, these drugs are relatively free of unwanted side effects. Examples of such non-steroidal anti-inflammatory compounds include aspirin, indomethacin, suprofen, cliprofen and ethyl 5-p-chlorobenzoyl-1, 4-dimethylpyrrole-2-acetate.

While many acylaminophenols, including the compounds employed in the treatment of the present invention, have been reported in the literature and used for a variety of purposes, to our knowledge, none have heretofore been reported to be useful as topical anti-inflammatory agents. A review of Chemical Abstracts has revealed the following disclosures relevant to pharmaceutical activities of related aminophenols as well as pharmaceutical and non-pharmaceutical activities of the substituted aminophenols specified below as being useful in the topical compositions and methods of the present invention. CA *57* 16492 (1962) contains a report of anti-rheumatic activity for N-nicotinyl-*p*-aminophenol and N-succinyl-*p*-aminophenol, and refers to Austrian Patent No. 222,647 (1962). *p*-Amino-phenol amides of sperm head oil acids and sperm blubber oil acids, mentioned in Netherlands Patent Application No. 6,505,431 as having hypocholesterolemic activity, are reported at CA *64* 14137 (1966). United States Patent No. 3,081,321 is reported at CA *60* 2833 (1964) to disclose analgesic activity of *p*-aminopenol amides of aliphatic monocarboxylic acids. Analgesic, anti-pyretic and anti-inflammatory activities are attributed to the dipropyl acetyl amide of p-aminophenol at CA *76* 153384x (1972), which refers to French M 7624 to Laboratories J. Berthier (1970); and the same activities are reported for the cystein derivative of *p*-aminophenol at CA *86* 107003f (1977), an abstract of an article by Portelli, Renzi, Cervato & Frigeni, Farmaco, Ed. Sci. 31 (11) 767—775 (1976). According to CA *86* 43215s (1977), Belgian Patent 834,304 reports that aryl amides of omega-aminoalkanoic acids, having the formula

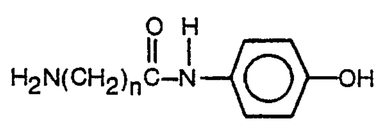

wherein n is 4, 5, 6 or 8, inhibit blood platelet aggregation. CA *89* 108193s (1978) has the same report with reference to British Patent 1,498,996, apparently a counterpart of the above Belgian patent. CA *88* 58568z (1978) reports that a Japanese patent publication (Kokai 77,110,835) discloses that certain benzanilide derivatives (none of which are 4-acylaminophenols, however) have shown anti-inflammatory activity when administered to mice orally or injected i.p.

Salicylaminophenol, which has the structure:

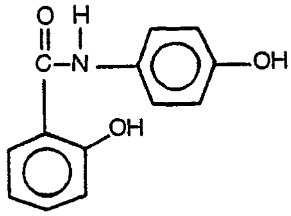

[also named 4'-hydroxysalicylanilide; N-(p-hydroxphenyl)salicylamide, and N-salicoylaminophenol], has been reported to have choleretic activity (Merck Index, Ninth Edition, 6724, page 894); fungistatic activity *in vitro* [CA *71* 69577b, (1969)]; activity in inhibiting allergic reaction or inflammation in mice when administered orally or i.p. [CA *88* 58568z, (1978)], molluscocidal and cercaricidal activity [CA *89* 192405t, (1978)]; a photosensitizing effect in guinea pigs [CA *83* 37501d, (1975)]; anthelminthic activity [CA *80* 133081a (1974)] and utility as an antioxidant for unsaturated rubber [CA *91* 6227e, (1979)].

For the acylaminophenols used in the composition and treatment of the present invention, having the formula:

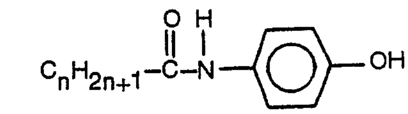

wherein n is an integer from 5 to 11, inclusive and closely relaed structural analogs (wherein n is less than 5 or greater than 11) the following activities have been reported:

| | |
|---|---|
| n=2 | analgesic [CA *74* 123508g, (1971)]; |
| n=2 or 3 | anti-inflammatory, antiedemic, analgesic, antipyretic and sedative — when combined with the enzymes, muramidase and amylase [CA *71* 42305z (1969) — refers to French M 5,575]; |
| n=3 | inceases biodegradability of polyolefins [CA *83* 194578b (1975) — refers to U.S. 3,903,029]; stabilizer for methyl methacrylate polymers [CA *77* 140970f (1972) — refers to Japan 72 07 629], |
| n=7 | local anesthetic [CA *50* 9424 refers to *Bull. Soc. Chim. France* (1955) 1603—9], |
| n=8,11 | polyester stabilizer [CA *76* 154779s (1972) refers to Ger. Offen. 2,036,712]; |
| n=3,8,11 | polyacetal stabilizer [CA *66* 29609d (1967) — refers to U.S. 3,288,885]; |
| n=9 | desensitizes thermoregulatory reflexes in guinea pigs [CA *72* 76596f (1970) — refers to J. Physiol. (London) *206* (3) 495—507(1970)]; |
| n=11 | thermal stabilizer in resins [CA *86* 107522z (1977) — refers to U.S. 4,002,701]; |
| n=15,2—15 | antioxidant [CA *58* 14257e (1963) — refers to Fr.1,309,355; CA *48* 1714i (1954) — refers to U.S. 2,654,722]. |

Disclosure of Invention

We have discovered in the course of investigations with experimentally induced inflammation that topical treatment with certain acylaminophenols results in unexpectedly improved suppression of inflammation. They also have local anesthetic properties, particularly when applied to mucous membrane as well as low toxicity in standard tests. The treatment of the present invention can also be used in the therapeutic treatment of a wide variety of dermatological and mucosal disorders in which inflammation is a component. Examples of these diseases as psoriasis, eczema, contact dermatitis and atopic dermatitis. Inflammation accompanying thermal or chemical burns, as well as sunburn are other areas for application of the therapy of this invention, as are diaper rash, insect bites, throat and eye inflammations, pruritus, poison ivy inflammation, hemorrhoids and inflammations caused by stoma adhesives and orthopedic casts. Also vesicular diseases, especially those characterized by acantholysis, and other blistering conditions, appear to be particularly susceptible to this therapy.

In accordance with the present invention, there is provided a composition adapted for only topical to the exclusion of oral and injectable, administration, for the treatment of an inflammatory condition, comprising a pharmaceutically acceptable topical vehicle containing an anti-inflammatory agent comprising an N-acylaminophenol containing a normal aliphatic side chain having from six to twelve carbon atoms, in an amount sufficient to combat said inflammatory condition.

The present invention also comprises use of an N-acylaminophenol containing a normal aliphatic side chain having from six to twelve carbon atoms for the manufacture of a medicament for the topical treatment of an inflammatory condition, for instance of the skin or mucous membrane.

Detailed Description of the Invention

The concentrations of the active ingredients in the composition will depend upon both the particular compounds used and upon the vehicle. When the vehicle used is propylene glycol/ethanol (30: 70 by

weight), the concentration of the active ingredients should be from about 0.01% to 10.0%, preferably from about 0.05% to about 5.0%. As indicated, lower or higher concentrations may be appropriate for different drugs and for different vehicles or delivery systems.

In addition to liquid vehicles, which may include sprays as well as nose, ear and eye drops, the topical vehicle may be a cream, lotion, gel or other form acceptable for topical use. Also suitable are throat lozenges, paints, sprays and pastilles. In general, treatment is effected by applying the composition or compositions to the affected area from one to about four times daily until the inflammation is relieved. The duration of treatment will vary with the severity of the condition.

TYPICAL FORMULATIONS (Unless otherwise indicated, throughout this specification, all amounts are by weight.)

A. *Topical Creams (oil in water emulsion type)*

| Ingredients | Conc. Range (By weight) |
|---|---|
| Acylaminophenol | 0.01—10.0 |
| Mineral oil | 3.0—10.0 |
| Cetyl alcohol | 1.0—5.0 |
| Isopropyl myristate | 1.0—5.0 |
| Polyoxyethylene (20) stearate | 1.0—5.0 |
| Propylene glycol | 1.0—60.0 |
| Butylated hydroxyanisole | 0—0.1 |
| Benzoic acid | 0.01—0.5 |
| Sodium edetate | 0.001—0.1 |
| Purified water | q.s. ad—100 |

In these oil-in-water emulsion creams, the cetyl alcohol may be replaced in whole or in part by other fats and waxes such as stearyl alcohol, glycerol monostearate, spermaceti or white petrolatum. Other emollients which may be used in place of or in addition to the mineral oil and isopropyl myristate include isopropyl palmitate, squalene, and hexadecyl alcohol. Surface-active agents other than polyoxyethylene (20) stearate may include other polyoxyethylene derivatives, sorbitan monoesters, polysorbates with suitable HLB values and other pharmaceutically acceptable surfactants known in the art. Other cosolvents which may be used in place of or in combination with the propylene glycol include glycerin, 1,2,6-hexanetriol, and the liquid polyethylene glycols (300;400). Preservatives other than benzoic acid include the parabens (methyl, propyl or combinations thereof) and sorbic acid.

Anti-oxidants other than butylated hydroxyanisole include butylated hydroxytoluene citric acid and propyl gallate, chelants other than sodium edetate include calcium disodium edetate and ethylenediamine tetraacetic acid (EDTA).

The following is a typical method of preparing the foregoing topical creams.

*Oil Phase.* Disperse mineral oil in isopropyl myristate in a suitable container (glass lined or stainless steel) heated to 80°—90°C, then add the oil-soluble ingredients such as butylated hydroxyanisole and a portion of the surfactant, and any fats or waxes from the general formula. Continue to heat with stirring until a uniform solution/melt is obtained.

*Aqueous Phase.* In a separate suitable container, heat the water to 80°—90°C. Then dissolve the benzoic acid and add the propylene glycol and remainder of the surfactant while mixing well.

*Emulsification Step.* Add the water phase to the oil phase at 80°—90° and agitate for a sufficient time period to insure complete mixing. Cool to the congealing point and add sufficient water to bring to the correct weight.

Incorporation of Active Ingredients

The acylaminophenol can be incorporated as finely divided micronized powder or dissolved in one of the vehicle ingredients.

B. *Solutions*

Liquid preparations for topical administration can be prepared in which the acylaminophenol is present in solution form, using pharmaceutically acceptable solvents. Other pharmaceutically acceptable

adjuvants, including a stabilizing system intended for maintaining the chemical integrity of the acylaminophenols, may also be included if warranted. The following general formulation is illustrative:

| Ingredients | Conc. Range (By weight) |
|---|---|
| Acylaminophenol | 0.01—10.0 |
| Adjuvant(s) | 0.0—0.2 |
| Solvent(s) | q.s. ad—100 |

The solvent system may contain one or a multiplicity of solvents suitable for topical administration, which may be selected from the lower alcohols (ethanol, isopropanol), glycols such as propylene, ethylene and polyethylene glycols (liquids at room temperature), other organic solvents such as dimethylsulfoxide, dimethylformamide, dimethylacetamide, 1,2,6-hexanetriol, butanediol and other liquid solvents completely or partially miscible with water.

The method of preparation for these solution forms of anti-inflammatory agents is generally (as is known in the art) to dissolve the solid ingredients in the primary solvent using a suitable container (glass or stainless steel lined) and mixers. The resulting solutions can then be filtered to remove extraneous matter and brought to final weight with the co-solvent and/or primary solvent, using the customary precautions to minimize loss of solvent by evaporation.

C. *Gels*

The above solutions can be made into semisolid (gel) preparations by the use of gelling agents such as hydroxypropyl cellulose, hydroxyethyl cellulose, carbomer polymers, and combinations of the above, according to the following general formula and procedure.

| Ingredients | Conc. Range (By weight) |
|---|---|
| Acylaminophenol | 0.01—10.0 |
| Alcohol | 10.0—70.0 |
| Co-solvent | 0—80.0 |
| Gelling agent | 0.5—5.0 |
| Purified water | q.s. ad—100 |

The gelling agent (e.g., hydroxypropyl cellulose) is added to the solution of acylaminophenol in its solvent system with agitation while avoiding clumping or excess air entrapment, using a glass-lined or stainless steel container.

D. *Ointments*

Ointments (water immiscible or wter miscible) of the compositions of the invention are typified by the following general formula.

| Ingredients | Conc. Range (By weight) |
|---|---|
| Acylaminophenol | 0.01—10.0% |
| Surfactant | 0—5.0% |
| Solvent | 1.0—20% |
| White petrolatum | q.s. ad—100 |

Typically, the ointment is prepared by heating the white petrolatum in a suitable container (glass-lined or stainless steel) until fluid and adding the active ingredients in any of the following forms: a) a suspension, in a finely powdered, micronized state, b) solubilized in a solvent system comprising such solvents as propylene glycol, polyethylene glycol 300, polyethylene glycol 400 or polyethylene glycol 1540 alone or in combination with 1,2,6-hexanetriol, propylene carbonate or other such solvents; with the acylaminophenol solubilized. Suitable oil soluble surfactants, for example, hydroxylated lanolin, ethoxylated lanolin derivatives or polyoxyethylene esters can be added into the petrolatum to make the ointment water miscible, or the surfactants may be omitted to make the ointment water immiscible.

E. *Powders*

The following is a typical dusting powder formulation containing an acylaminophenol in accordance with the invention.

| Ingredients | Conc. Range (By weight) |
|---|---|
| Acylaminophenol | 0.01—10.0 |
| Talcum powder | 0—99.99 |
| Cornstarch | 0—99.99 |

The acylaminophenol is mixed with the talcum powder and/or cornstarch by adding the latter, preferably in geometric progressions, until the entire mixture is blended uniformly, using a glass-lined or stainless steel container and suitable mixing equipment. Particle size distributions of the acylaminophenol in the range of 5—25 µm (microns) are preferred to facilitate a homogeneous mixture.

F. *Aerosol*

Many types of aerosol formulations can be used as vehicles for the acylaminophenols in accordance with the present invention. These can vary with the type of propellant and concentrate used in the manufacture, as is known to those skilled in the art. The example given below is for a general formula for a quick breaking alcoholic foam.

| Ingredients | Conc. Range (By weight) |
|---|---|
| Concentrate Ethoxylated lanolin alcohol | 0.5—2.5 |
| Cetyl alcohol | 0.5—2.5 |
| Alcylaminophenol, micronized | 0.1—10.0 |
| Ethanol, USP | 30.0—90.0 |
| Distilled water | 9.49—55.0 |
| Final Product | |
| Concentrate | 80.0—5.0 |
| Propellants 12/114,40:60 | 5.0—20.0 |

The acylaminophenol is either dissolved or dispersed in the ethanol/water solvent system together with the cetyl alcohol and the lanolin derivative. A preservative (e.g., Hyamine 1622) can be added if warranted. The concentrate is then mixed with the propellant, either by cold filling or by pressure filling according to known methods of manufacture.

G. *Impregnated Tape*

An adhesive tape impregnated with an acylaminophenol of the invention can be prepared using the technology and directions stated in Patent No. 3,632,740 issued on January 4, 1972 to R. C. V. Robinson et al. ("Topical Device for the Therapeutic Management of Dermatological Lesions With Steroids"). The acylaminophenol can be added in solution in an appropriate organic solvent.

As indicated above, the acylaminophenol compounds employed in the composition and treatment of the present invention are known compounds. Suitable methods for their preparation are described, for example, in *Helvetica Chimica Acta 22*: 82—112 (1939), more particularly at pages 89—93.

Generally, *p*-aminophenol is reacted with the appropriate acid anhydride or chloride.

H. *Throat Lozenge* — Candy Base

Micronized acylaminophenol .01—0.2 gm.

Candy Base Q.S. —2.0 gm.

The candy base is composed of sucrose and corn syrup solids, food color and flavor.

Method of Preparation:

1. The candy base is prepared by metering 345 pounds (46 gallons) of Liquid Sugar No. 1 and 230 lbs. of 43° Baumé Corn Syrup into a steam jacketed kettle known as a premelter.

2. The mass is cooked to 238°F. under atmospheric pressure and then pumped into a storage vessel which feeds a National Equipment Continuous Cooker. As the syrup passes through a coil in the cooking chamber, which is surrounded by high pressure steam, it reaches a temperature of 290—300°F.

3. Batches are delivered to a stainless steel receiving kettle maintained at 28—29 inches of vacuum by means of multi-stage, steam vacuum ejectors for a period of 7 minutes. The vacuum serves to remove additional moisture without additional heat.

4. At this stage, the candy base is composed of approximately:
64.77% Sugar Solids
34.63% Corn Syrup Solids
0.6% Moisture.

5. 300 pounds of candy base is then transformed (150 pounds to each of two), stainless steel mixing kettles, one designated as Part "A" of the batch and the other Part "B".

6. Approved food color Q.S. is divided in two equal parts for mixing with Parts "A" and "B".
Flavor Compound Q.S. is divided in two equal parts for mixing with Parts "A" and "B".
Micronized acylaminophenol to yield 0.5 to 10% of the lozenge is divided into two equal parts for mixing with Parts "A" and "B".

7. The batch is then placed in an automatic batch roller for feeding a Hansella type forming machine which punches out the lozenges. The lozenges are fed through a cooling and hardening conveyor in a dehumidified room. They are then transferred to machines for individual wrapping.

I. *Acylaminophenol — Lozenge, Sucrose Base*

| | |
|---|---|
| Acylaminophenol | 0.02—0.06 gm. |
| Pluronic F68 | 0.40—0.60 gm. |
| Sucrose | 2 gm. |
| Binder Solution Q.S. | |
| Flavor Q.S. | |
| Lubricant Q.S. | |

Method of Preparation

1. Acylaminophenol is dissolved in molten Pluronic F68. The solution is allowed to cool to the solid state and is subsequently pulverized and sieved through #40 mesh screen.
2. Sucrose is pulverized and sieved through a #40 mesh screen.
3. #1 and #2 are mixed then are wet granulated with an appropriate aqueous binder solution, e.g. gum tragacanth, acacia, or methyl cellulose or other binder well known in the art.
4. Flavor, e.g., peppermint oil, plus menthol or cherry flavor are added and mixed.
5. The wet granules are dried in a hot air oven.
6. An appropriate lubricant, e.g., talc or magnesium stearate or stearic acid is added to the dry granulation and mixed.
7. The granulation is compressed in a tablet machine to produce tablets for slow dissolution in the mouth.

As an alternative to the above described (wet granulation) procedure dry granulation by "slugging" or chilsonating may be substituted.

J. *Acylaminophenol Pastille*

| | |
|---|---|
| Acylaminophenol | 0.01—0.2 gm. |
| Pastille Base | Q.S.—2.0 gm. |

The base may be composed of glycogelatin or other suitable preparations containing gelatin and glycerin, or a mixture of acacia and sucrose. Glycerin in the base may be substituted with propylene glycol.

K. *Acylaminophenol Throat Paint*

Acylaminophenol (0.5—10%) is dissolved in propylene glycol or glycerin. The final concentration in glycerin or propylene glycol will constitute a saturated solution of acylaminophenol and will vary according to the solubility of the particular acylaminophenol selected, but will provide 0.5—10% base may be substituted with propylene glycol.

L. *Acylaminophenol Throat Spray*

| | |
|---|---|
| Acylaminophenol | 0.5—10% |
| Flavor | Q.S. |
| Saccharin Sodium | Q.S. |
| Alcohol 50% | Q.S.—100 |
| Propellant | Q.S. |

Propellant may be selected from available propellants known in the art, e.g., chlorofluorocarbons (propellant 114 or propellant 12 or mixtures thereof), $CO_2$, nitrogen with appropriate head space.

Alcohol, 50%, may be substituted with propylene glycol, glycerin or mixtures of alcohol, glycerin and propylene glycol to provide a 0.50—10% solution of the particular acylaminophenol selected.

Example 1

The effect of selected compositions on cutaneous inflammation caused by topical application of arachidonic acid was evaluated. In addition to inflammation, the proliferative response of the epidermis treated with arachidonic acid and the compositions was evaluated.

Fourteen acylaminophenols were studied. The drugs were dissolved in propylene glycol plus ethanol (30:70) and were used at 1% concentrations unless otherwise stated. Fourteen groups of five guinea pigs each served as the experimental subjects. The dorsal skin of both ears was treated once daily for four days (9.00 am) with 25 µl 1% solution of AA in propylene glycol/ethanol (30/70). Immediately after this application, one ear of each guinea pig was randomly chosen for application of 25 µl of the test compound, while the contralateral ear received the vehicle, alone. During the four-day experimental period, DNA synthesized was labeled by continuous administration of a radioactive percursor, $^3$H-thymidine (3 i.p. injections/day of 10 µCi each). Erythema developing in response to topical AA was measured daily on a subjective scale of 0 to 3; where 0 = none, 1 = mild, 2 = moderate, and 3 = intense. The average erythema scores were plotted as a function of time, and areas under the response curves were determined for expression of *total erythema*. On the fifth day, animals were killed and six mm diameter cicrular samples of dorsal skin from the central portion of the treatment sites were harvested and solubilized in NCS® solubilizer. These tissue solutions were acidified and a simple flour (diphenyloxazole in toluene) was added for determination of radioactivity by liquid scintillation counting. Radioactivity expressed as counts-per-minute (cpm) correlate with the amount of proliferate activity in the epidermis.

Results

It was found that topical application of acetamidophenol (acetaminophen) had no significant effect on AA-induced erythema or proliferation. The appliation of 4-propionylaminophenol, or 4-n-tetradecanoylaminophenol or 4-n-hexadecanoylaminophenol was also without substantial effect. Benzoylaminophenol, an example of a 4-acylaminophenol of an aromatic acid was also inactive in this model.

It was found that 4-n-butyrolaminophenol significantly inhibited AA-induced epidermal proliferation, but was without effect on erythema.

The acylaminophenols with normal aliphatic side chains ranging from six to twelve carbon atoms showed good activity against both AA-induced erythema and proliferation. These included 4-n-hexanoylaminophenol, 4-n-decanoylaminophenol, 4-n-undecanoylaminophenol, and 4-n-dodecanoylaminophenol, 4-n-heptanoylaminophenol, 4-n-octanoylaminophenol, and 4-n-nonanoylaminophenol.

In addition, the 4-acylaminophenol of salicylic acid (4-salicylaminophenol) showed activity against both erythema and proliferation. These data are summarized in Table 1.

TABLE 1 — Activity of topical acylaminophenols against arachidonic acid-induced epidermal poliferation and erhythema in guinea pig skin.

| Material Tested | cpm S.E. | % Reduction | Total Erythema | % Reduction |
|---|---|---|---|---|
| 1% Acetaminophen | 3140 ± 346 | 8.0 | 5.2 | 0 |
| Vehicle control | 3415 ± 247 | | 5.2 | |
| 1% 4-propionylaminophenol | 1777 ± 251 | 2.6 | 5.5 | −14.6 |
| Vehicle control | 1825 ± 160 | | 4.5 | |
| 1% 4-*n*-butyroylaminophenol | 2680 ± 360 | 27.8* | 4.6 | 4.2 |
| Vehicle control | 3716 ± 343 | | 4.8 | |
| 1% 4-*n*-hexanoylaminophenol | 1488 ± 104 | 28.3* | 1.9 | 50.0 |
| Vehicle control | 2076 ± 158 | | 3.8 | |
| 1% 4-*n*-heptanoylaminophenol | 1435 ± 77 | 27.3* | 1.8 | 47.0 |
| Vehicle control | 1973 ± 211 | | 3.4 | |
| 1% 4-*n*-octanoylaminophenol | 1589 ± 361 | 39.1* | 2.7 | 49.0 |
| Vehicle control | 2611 ± 164 | | 5.3 | |
| 1% 4-*n*-nonanoylaminophenol | 1472 ± 135 | 20.9* | 2.1 | 34.4 |
| Vehicle control | 1860 ± 214 | | 3.2 | |
| 1% 4-*n*-decanoylaminophenol | 1311 ± 48 | 37.5+ | 2.0 | 47.4 |
| Vehicle control | 2098 ± 409 | | 3.8 | |
| 1% 4-*n*-undecanoylaminophenol | 1380 ± 205 | 37.3* | 2.1 | 38.2 |
| Vehicle control | 2202 ± 262 | | 3.4 | |
| 1% 4-*n*-dodecanoylaminophenol | 2487 ± 368 | 29.0* | 3.8 | 29.0 |
| Vehicle control | 3504 ± 328 | | 5.6 | |
| 1% 4-*n*-tetradecanoylaminophenol | 1681 ± 296 | 15.1 | 3.8 | 0.0 |
| Vehicle control | 1980 ± 237 | | 3.8 | |
| 0.56% 4-*n*-hexadecanoylamiophenol | 3275 ± 463 | 15.1 | 4.8 | 14.3 |
| Vehicle control | 3857 ± 329 | | 5.6 | |
| 1% 4-benzoylaminophenol | 2214 ± 316 | 4.2 | 3.8 | 20.8 |
| Vehicle control | 2312 ± 409 | | 4.8 | |
| 1% 4-salicylaminophenol | 1532 ± 91 | 23.3* | 3.2 | 41.8 |
| Vehicle control | 1998 ± 172 | | 5.5 | |

*Statistically significant reduction in proliferation
±Not significant due to variability

Having thus described our invention, what we desire to secure by Letters Patent is defined in the appended claims.

**Claims**

1. Use of an N-acylaminophenol containing a normal aliphatic side chain having from six to twelve carbon atoms for the manufacture of a medicament for the topical treatment of an inflammatory condition.

2. The use according to Claim 1 wherein said acylaminophenol is 4-*n*-hexanoylaminophenol, 4-*n*-heptanoylaminophenol, 4-*n*-octanoylaminophenol, 4-*n*-nonanoylaminophenol, 4-*n*-n-decanoylaminophenol, 4-*n*-undecanoylaminophenol or 4-*n*-dodecanoylaminophenol.

3. A composition adapted for only topical, to the exlusion of oral and injectable, administration, for the treatment of an inflammatory condition, comprising a pharmaceutically acceptable topical vehicle containing an anti-inflammatory agent comprising an N-acylaminophenol containing a normal aliphatic side chain having from six to twelve carbon atoms, in an amount sufficient to combat said inflammatory condition.

4. A composition according to claim 3 wherein said acylaminophenol is 4-pn-hexanoylamino-phenol, 4-*n*-heptanoylaminophenol, 4-*n*-octanoylaminophenol, 4-*n*-undecanoylaminophenol or 4-*n*-dodecanoylaminophenol.

5. A composition according to Claim 3 or 4 wherein said acylaminophenol is present in said vehicle in an amount of from 0.01% to about 10.0% by weight.

6. A composition according to any of Claims 3 to 5 wherein said vehicle comprises propylene glycol.

7. A composition according to Claim 6 wherein said vehicle comprises a mixture of propylene glycol and ethanol.

8. A composition according to Claim 6 wherein said vehicle comprises a mixture of propylene glycol and dimethylsulfoxide.

9. A method of producing a composition according to any one of claims 3 to 8, comprising mixing an amount of an N-acylaminophenol sufficient to combat an inflammatory condition with a pharmaceutically acceptable topical vehicle.

**Patentansprüche**

1. Verwendung eines eine gerade aliphatische Seitenkette mit sechs bis zwölf Kohlenstoffatomen enthaltenden N-Acylaminophenols für die Herstellun eines Medikaments für die topische Behandlung eines Entzündungszustandes.

2. Verwendung nach Anspruch 1, worin das genannte Acylaminophenol 4-*n*-Hexanoylaminophenol, 4-*n*-Heptanolaminophenol, 4-*n*-Octanoylaminophenol, 4-*n*-Nonanoylaminophenol, 4-*n*-Decanoylaminophenol, 4-*n*-Undecanoylaminophenol oder 4-*n*-Dodecanoylaminophenol ist.

3. Eine nur für topische, unter Ausschluß einer oralen und injizierbaren Verabreichung, angepaßte Zusammensetzung für die Behandlung eines Entzündungszustandes, umfassend eines pharmazeutisch anehmbaren topischen Träger mit einem Gehalt an einem ein N-Acylaminophenol, das eine gerade aliphatische Seitenkette mit sechs bis zwölf Kohlenstoffatomen enthält, umfassenden entzündungshemmenden Mittel in einer zur Bekämpfung des Entzündungszustandes ausreichenden Menge.

4. Zusammensetzung nach Anspruch 3, worin das Acylaminophenol 4-*n*-Hexanoylphenol, 4-*n*-Heptanoylaminophenol, 4-*n*-Octanoylaminophenol, 4-*n*-Undecanoylaminophenol oder 4-*n*-Dodecanoylaminophenol ist.

5. Zusammensetzung nach Anspruch 3 oder 4, worin das Acylaminophenol in dem Träger in einer Menge von 0,01 — etwa 10,0 Gew.-% vorliegt.

6. Zusammensetzung nach einem der Ansprüche 3 bis 5, worin der Träger Propylenglycol umfasst.

7. Zusammensetzung nach Anspruch 6, worin der Träger ein Gemisch aus Propylenglycol und Ethanol umfasst.

8. Zusammensetzung nach Anspruch 6, worin der Träger ein Gemisch aus Propylenglycol und Dimethylsulfoxid umfasst.

9. Verfahren zur herstellung einer Zusammensetzung nach einem der Ansprüche 3 bis 8, umfassend ein Vermischen einer zur Bekämpfung eines Entzündungszustandes aureichenden Menge eines N-Acylaminophenols mit einem pharmazeutisch annehmbaren topischen Träger.

**Revendications**

1. Utilisation d'un N-acylaminophénol contenant une chaîne latérale aliphatique normale ayant de 6 à 12 atomes de carbone servant à la fabrication d'un médicament pour traitement topique d'un état inflammatoire.

2. Utilisation selon la revendication 1, dans laquelle ledit acylaminophénol est le 4-n-hexanoylaminophénol, 4-n-heptanoylaminophénol, 4-n-octanoylaminophénol, 4-n-nonanoylaminophénol, 4-n-décanoylaminophénol, 4-n-undecanoylaminophénol ou 4-n-dodecanoylaminophénol.

3. Composition destiné au seul traitement topique à l'exclusion d'une administration orale et injectable pour le traitement d'un état inflammatoire, comprenant un véhicule topique pharmaceutiquement acceptable qui contient un agent anti-inflammatoire comprenant un N-acylaminophénol qui contient une chaîne latérale aliphtique normale de 6 à 12 atomes de carbone, en une quantité suffisante pour combattre ledit état inflammatoire.

4. Composition selon la revendication 3, dans laquelle ledit acylaminophénol est le 4-n-hexanoylaminophénol, 4-n-heptanolaminophénol, 4-n-octanoylaminophénol, 4-n-undecanoylaminophénol ou 4-n-dodecanoylaminophénol.

5. Composition selon la revendication 3 ou 5, dans laquelle ledit acylaminophénol est présent dans ledit véhicule en une quantité de 0,01% à environ 10,0% en poids.

6. Composition selon l'une quelconque des revendications 3 a 5, dans laquelle ledit véhicule comprend du propylèneglycol.

7. Composition selon la revendication 6, dans laquelle ledit véhicule comprend un mélange de propylèneglycol et d'éthanol.

8. Composition selon la revendication 6, dans laquelle ledit véhicule comprend un mélange de propylèneglycol et de diméthylsulfoxyde.

9. Procédé de fabrication d'une composition selon l'une quelconque des revendications 3 à 8, qui consiste à mélanger un N-acylaminophénol en une quantité suffisante pour lutter contre un état inflammatoire avec un véhicule topique pharmaceutiquement acceptable.